# EUROPEAN PATENT APPLICATION

(11) **EP 4 772 133 A1**
(43) Date of publication of application: **08.07.2026**
(21) Application number: 23951968.9
(22) Date of filing: 13.10.2023
(51) Int. Cl.: A61B 34/35, A61B 34/30

(54) **PARALLEL MOTION MECHANISM, SURGICAL INSTRUMENT, AND SURGICAL ROBOT**

(30) Priority: 13.09.2023 CN 202311178626
(71) Applicant: Cornerstone Technology (Shenzhen) Limited, Shenzhen Guandong (CN)
(72) Inventor: HUANG, Qun, Shenzhen, Guangdong 518066 (CN)
(74) Representative: Balder IP Law, S.L.
(86) International application number: PCT/CN2023/124465
(87) International publication number: WO 2025/055042

(57) **Abstract**

A parallel motion mechanism (130), a surgical instrument (100), and a surgical robot (200) are provided. The parallel motion mechanism (130) includes a proximal joint assembly (10), a distal joint assembly (20), a plurality of constraint wires (30), and a plurality of driving wires (40). The proximal joint assembly (10) includes a first, second, and third proximal joint portions (11, 12, 13). The second proximal joint portion (12) swings relative to the first proximal joint portion (11) along a second plane (P2), and the third proximal joint portion (13) swings relative to the second proximal joint portion (12) along a first plane (P1). The first and second planes (P1, P2) intersect at central axis (PC) of the parallel motion mechanism. The distal joint assembly (20) includes first, second, and third distal joint portions (21, 22, 23). The third distal joint portion (23) is fixed relative to the third proximal joint portion (13). The second distal joint portion (22) swings relative to the third distal joint portion (23) along the first plane (P1), and the first distal joint portion (21) swings relative to the second distal joint portion (22) along the second plane (P2). Each constraint wire (30) has two ends fixed to the first proximal joint portion (11) and the first distal joint portion (21). Each driving wire (40) has two ends fixed to the first distal joint portion (21) and a transmission device.

## Description

### CROSS-REFERENCE TO RELATED APPLICATIONS

The present application claims priority to Chinese Patent Application No. 202311178626.7, filed on September 13, 2023, entitled "PARALLEL MOTION MECHANISM, SURGICAL INSTRUMENT, AND SURGICAL ROBOT", and the entire contents of which are incorporated herein by reference.

### TECHNICAL FIELD

The present disclosure relates to the technological field of medical devices, and more particularly, to a parallel motion mechanism for a surgical instrument, the surgical instrument including the parallel motion mechanism, and a surgical robot including the surgical instrument.

### BACKGROUND

In related arts, a parallel motion mechanism is coupled between an end effector and a rear end of a surgical instrument for moving the end effector of the surgical instrument to easily reach a desired operating position without changing the orientation of the end effector. Compared to a joint-series structure, the use of the parallel motion mechanism ensures a larger range of motion for the end effector.

### SUMMARY

A series of simplified concepts are introduced in the summary, which will be further explained in the detail description. The Summary of the present disclosure does not intend to limit the key or essential features of the claimed technical solution, and also no intended to determine the scope of protection of the claimed technical solution.

A first aspect of an embodiment of the present disclosure provides a parallel motion mechanism of a surgical instrument, including:
a proximal joint assembly including a first proximal joint portion, a second proximal joint portion, and a third proximal joint portion coupled in sequence, the third proximal joint portion being configured to swing along a first plane relative to the second proximal joint portion, the second proximal joint portion being configured to swing along a second plane relative to the first proximal joint portion, and the first plane and the second plane intersecting with each other at a central axis of the parallel motion mechanism;
a distal joint assembly being configured to couple to an end effector, and including a first distal joint portion, a second distal joint portion, and a third distal joint portion coupled in sequence, the third distal joint portion coupled to the third proximal joint portion and fixed relative to the third proximal joint portion, the second distal joint portion being configured to swing along the first plane relative to the third distal joint portion, and the first distal joint portion being configured to swing along the second plane relative to the second distal joint portion;
a plurality of constraint wires being configured to maintain an orientation of the first distal joint assembly relative to the first proximal joint, each of the plurality of constraint wires having an end fixed to the first proximal joint portion and another end fixed to the first distal joint portion; and
a plurality of driving wires being configured to actuate the distal joint, each of the plurality of driving wires having an end fixed to the first distal joint portion and another end configured to couple to a rear-end transmission device, each of the plurality of driving wires having a portion routed through the parallel motion mechanism, and a sum of lengths of the portions of the plurality of driving wires remaining unchanged.

Optionally, each of the plurality of driving wires has a section routed through the proximal joint assembly and a section routed through the distal joint, both of the sections of each of the plurality of driving wires are routed parallel to the central axis when the parallel motion mechanism is in a neutral state. The plurality of driving wires includes:
a first pair of driving wires, where a section of each driving wire in the first pair of driving wires routed through the proximal joint assembly and a section of the driving wire routed through the distal joint assembly are located at two opposite sides of the first plane, respectively, and are equidistant from the first plane; and
a second pair of driving wires, where a section of each driving wire in the second pair of driving wires routed through the proximal joint assembly and a section of the driving wire routed through the distal joint assembly are located at two opposite sides of the second plane, respectively, and are equidistant from the second plane.

Optionally, the first pair of driving wires includes a first driving wire and a second driving wire. The first driving wire and the second driving wire are configured to drive the first distal joint portion to swing along the second plane relative to the second distal joint portion. A section of the first driving wire routed through the proximal joint assembly and a section of the second driving wire routed through the proximal joint assembly are located at two opposite sides of the first plane, respectively.

Optionally, the section of the first driving wire routed through the proximal joint assembly and the section of the second driving wire routed through the proximal joint assembly are equidistant from the first plane.

Optionally, the first driving wire and the second driving wire each has a section routed straightly between the third distal joint portion and the third proximal joint portion and parallel to the second plane. The sections of the first driving wire and the second driving wire are located at two opposite sides of the second plane, respectively.

Optionally, the second pair of driving wires include a third driving wire and a fourth driving wire. The third driving wire and the fourth driving wire are configured to drive the second distal joint portion to swing along the first plane relative to the third distal joint portion. A section of the third driving wire routed through the proximal joint assembly and a section of the fourth driving wire routed through the proximal joint assembly are located at two opposite sides of the second plane, respectively.

Optionally, the section of the third driving wire routed through the proximal joint assembly and the section of the fourth driving wire routed through the proximal joint assembly are equidistant from the second plane.

Optionally, the third driving wire and the fourth driving wire each is parallel to the first plane. The sections of the third driving wire and the fourth driving wire are located at two opposite sides of the first plane, respectively.

Optionally, each of the plurality of driving wires has a section routed straightly between the third distal joint portion and the third proximal joint portion, the sections of the plurality of driving wires are routed along different straight lines on a hyperboloid of one sheet. A principal axis of the hyperboloid of one sheet coincides with the central axis of the parallel motion mechanism.

Optionally, the plurality of constraint wires includes at least two pairs of constraint wires; the at least two pairs of constraint wires are routed parallel to the central axis, and are symmetrical with respect to each of the first plane and the second plane when the parallel motion mechanism is in a neutral state.

Optionally, the parallel motion mechanism further includes a sleeve. The sleeve has two ends coupled to the third distal joint portion and the third proximal joint portion, respectively. Each of the plurality of driving wires and the plurality of constraint wires is routed through an interior of the sleeve.

A second aspect of an embodiment of the present disclosure provides a surgical instrument, including:
a parallel motion mechanism according to any above solution;
an end effector coupled to the distal joint assembly of the parallel motion mechanism; and
a rear-end transmission device, each of the plurality of driving wires having a rear end coupled to the rear-end transmission device.

Optionally, the end effector is a hook, a spatula, a needle, a clamp, or a pair of scissors.

Optionally, the surgical instrument further includes an end joint assembly. The end effector is coupled to the distal joint through the end joint assembly.

Optionally, the end joint assembly includes a yaw joint base and a pitch joint base. The end effector has a proximal end coupled to a distal end of the yaw joint base and rotatable about a yaw axis relative to the yaw joint base. The yaw joint base has a proximal end coupled to a distal end of the pitch joint base and rotatable about the yaw axis relative to the pitch joint base.

Optionally, the proximal end of the end effector is rotatably coupled to the distal end of the yaw joint base through a first clevis pin, and/or the proximal end of the yaw joint base is rotatably coupled to the distal end of the pitch joint base through a second clevis pin.

Optionally, the end effector includes a first jaw and a second jaw. Each of the first jaw and the second jaw has a proximal end pivotably coupled to the yaw joint base through the first clevis pin. The first jaw and the second jaw are separately pivotable relative to the yaw joint base about the first clevis pin.

Optionally, the surgical instrument further includes at least one pair of end joint driving wires. The at least one pair of end joint driving wires is configured to actuate the end joint assembly relative to the distal joint assembly, thereby moving the end effector relative to the distal joint assembly. The at least one pair of end driving wires is routed at the first plane and/or the second plane and parallel to the central axis when the parallel motion mechanism is in a neutral state. Each of the at least one pair of end driving wires has an end coupled to the rear-end transmission device.

Optionally, the surgical instrument further includes an electric cable. The electric cable is electrically coupled to the end effector. The electric cable is routed along the central axis when the parallel motion mechanism is in a neutral state.

A third aspect of an embodiment of the present disclosure provides a surgical robot, including:
a robotic arm equipped with an instrument transmission device; and
a surgical instrument according to any above solution, the surgical instrument removably coupled to the robotic arm, and the instrument transmission device being engaged with the rear-end transmission device to actuate the rear-end transmission device to pull in or release the plurality of driving wires.

The details of one or more embodiments of the present disclosure are set forth in the accompanying drawings and descriptions below. Other features, objectives, and advantages of the present disclosure will become apparent from the specification, the drawings, and the claims.

### DESCRIPTION OF DRAWINGS

The following drawings are incorporated as part of the present disclosure for the understanding purpose. The drawings illustrate the embodiments and descriptions of the present disclosure, and serves to explain the principles of the present disclosure.

In the attached drawings:
FIG. 1 illustrates a top schematic view of a surgical robot according to an embodiment of the present disclosure.
FIG. 2 illustrates a side schematic view of a robotic arm system in FIG. 1.
FIG. 3 illustrates a schematic view of a surgical instrument according to an embodiment of the present disclosure.
FIG. 4 illustrates a schematic view of the surgical instrument in FIG. 3, where a sleeve thereof is omitted.
FIG. 5 illustrates a schematic view of some components of the surgical instrument in FIG. 3, where end joint driving wires are shown.
FIG. 6 illustrates a schematic view of a parallel motion mechanism according to an embodiment of the present disclosure, where the sleeve and parallel motion driving wires thereof are omitted.
FIG. 7 illustrates a schematic view of the parallel motion mechanism according to an embodiment of the present disclosure, where the sleeve and constraint wires thereof are omitted, and the parallel motion mechanism is in a zero-position state.
FIG. 8 illustrates a side schematic view of the parallel motion mechanism in FIG. 7 taken along a direction parallel to a first plane, where the sleeve and the constraint wires thereof are omitted, and a first proximal joint and a first distal joint are deviated from a zero-position angle.
FIG. 9 illustrates a side schematic view of the parallel motion mechanism in FIG. 7 taken along a direction parallel to a second plane, where the sleeve and the constraint wires are omitted, and the first distal joint and a second distal joint are deviated from the zero-position angle.
FIG. 10 illustrates a schematic view taken along L1 direction in FIG. 5.
FIG. 11 illustrates a schematic view taken along L2 direction in FIG. 5.
FIG. 12 illustrates a cross-sectional view taken along line D-D in FIG. 7.
FIG. 13 illustrates a schematic view of a proximal joint assembly in FIG. 3.
FIG. 14 illustrates a partial enlarged view in FIG. 3, where an end effector and an end joint assembly are shown.

**Description of numerals for the drawings:**

| | | | |
|---|---|---|---|
| 10: | proximal joint assembly | 60B: | proximal through holes for parallel motion driving wires |
| 11: | first proximal joint portion | 60A: | distal through holes for parallel motion driving wires |
| 12: | second proximal joint portion | 61B: | first proximal through hole |
| 13: | third proximal joint portion | 61A: | first distal through hole |
| 14: | second tooth | 62B: | second proximal through hole |
| 14A: | surface of the second tooth | 62A: | second distal through hole |
| 15: | first engagement recess | 63B: | third proximal through hole |
| 16: | second engagement recess | 63A: | third distal through hole |
| 17: | first tooth | 64B: | fourth proximal through hole |
| 17A: | surface of first tooth | 64A: | fourth distal through hole |
| 18: | second rolling surface | 70B: | proximal through holes for constraint wires |
| 19: | first rolling surface | 70A: | distal through holes for constraint wires |
| 20: | distal joint assembly | 71B: | first proximal constraint wire through hole |
| 21: | first distal joint portion | 71A: | first distal constraint wire through hole |
| 22: | second distal joint portion | 72B: | second proximal constraint wire through hole |
| 23: | third distal joint portion | 72A: | second distal constraint through hole |
| 30: | constraint wires | 73B: | third proximal constraint wire hole |
| 31: | first constraint wire | 73A: | third distal constraint wire through hole |
| 32: | second constraint wire | 74B: | fourth proximal constraint wire through hole |
| 33: | third constraint wire | 74A: | fourth distal constraint wire through hole |
| 34: | fourth constraint wire | 80B: | proximal through holes for end joint driving wires |
| 40: | driving wires | 80A: | distal through holes for end joint driving wires |
| 41: | first driving wire | 81B: | first proximal driving wire through hole |
| 42: | second driving wire | 81A: | first distal driving wire through hole |
| 43: | third driving wire | 82B: | second proximal driving wire through hole |
| 44: | fourth driving wire | 82A: | second distal driving wire through hole |
| 47: | first pair of driving wires | 83B: | third proximal driving wire through hole |
| 48: | second pair of driving wires | 83A: | third distal driving wire through hole |
| 50: | end joint driving wires | 84B: | fourth proximal driving wire through hole |
| 51: | first end joint driving wire | 84A: | fourth distal driving wire through hole |
| 52: | second end joint driving wire | 200: | surgical robot |
| 53: | third end joint driving wire | 210: | control system |
| 54: | fourth end joint driving wire | 220: | robotic arm system |
| 91: | sleeve | 221: | robotic arm |
| 92: | cable passage | 222: | instrument holder |
| 100: | surgical instrument | 230: | imaging system |
| 110: | end effector | A1: | first proximal axis |
| 111: | first jaw | A3: | third proximal axis |
| 112: | second jaw | B1: | second proximal axis |
| 120: | end joint assembly | B3: | fourth proximal axis |
| 121: | yaw joint base | P1: | first plane |
| 122: | pitch joint base | P2: | second plane |
| 123: | first clevis pin | PC: | central axis |
| 124: | second clevis pin | X1: | yaw axis |
| 130: | parallel motion mechanism | X2: | pitch axis |
| 140: | shaft | DA: | axial direction |
| 150: | rear-end transmission device | | |

### DETAILED DESCRIPTION

In the following description, specific details are provided to facilitate thorough understanding of the present disclosure. However, it is obvious to those skilled in the art that the present disclosure can be implemented without one or more of these details. In other instances, technical features well known in the art are not described to avoid confusion with the present disclosure.

To thoroughly understand the present disclosure, detailed descriptions will be provided as follows. It should be understood that embodiments are provided to make the disclosure of the present disclosure thorough and complete, and to fully convey the concept of the present disclosure to those ordinary skilled in the art. Obviously, the implementation of the embodiments of the present disclosure is not limited to specific details familiar to those skilled in the art. The preferred embodiments of the present disclosure are described as follows, however, the present disclosure may also have other embodiments in addition to these detailed descriptions.

The ordinal terms such as "first" and "second" cited in the present disclosure are merely identifiers without any other specific meanings such as specific orders. Furthermore, for example, a term "first component" does not inherently imply the existence of a "second component", and similarly, the term "second component" does not inherently imply the existence of the "first component". The use of the terms such as "first", "second", and "third" does not indicate any order, and these terms can be interpreted as designations.

It should be noted that the terms "on", "below", "front", "back", "left", "right", "inside", "outside" and similar expressions used in the present disclosure are only for illustrative purposes but not for limiting purposes.

The terms "distal end" and "proximal end" used in the present disclosure serve as directional terms, which are commonly used in the field of interventional medical devices. Where, "distal end" refers to an end that is farther away from the operator during the surgical procedure, and "proximal end" refers to an end that is closer to the operator during the surgical procedure.

The terms "parallel", "perpendicular", and similar expressions used in the present disclosure encompass absolute parallel and perpendicular relationships, and also approximate parallel and perpendicular relationships (e.g., within a range of -5° to +5° from absolute parallel or perpendicular relationships), which may have equivalent effects.

The term "length remains unchanged" and similar expressions used in the present disclosure means that the original length is maintained or fluctuated within a certain range. For example, any variation within ±5% of the original length falls within the scope described by the term "length remains unchanged", and may have equivalent effects.

The term "rigid material" used in the present disclosure refers to a material with good resistance to deformation, which may have minimal or negligible deformation under an external force.

Implementations of the present technology will now be described, by way of example only, with reference to the attached figures. The figures illustrate preferred embodiments of the present disclosure, and do not limit the present disclosure.

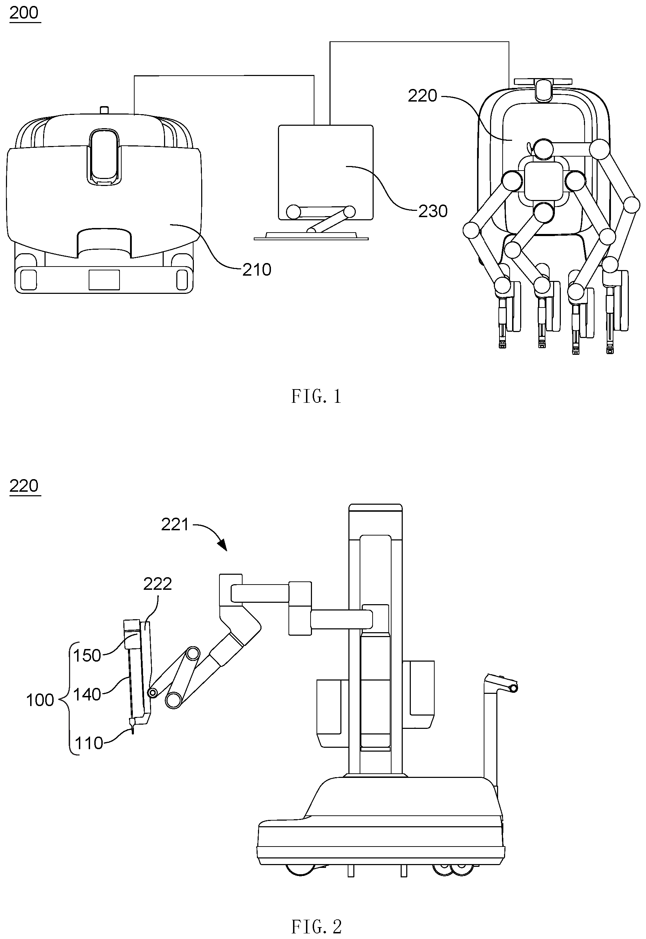

A surgical robot 200, which is provided according to an embodiment of the present disclosure, is a robot that can be remotely manipulated to complete surgeries. Referring to FIG. 1, the surgical robot 200 may include a control system 210 (also referred to as a surgeon console 210), a robotic arm system 220 (also referred to as a patient-side robotic arm system 220), and an imaging system 230 (also referred to as an endoscope system 230).

The control system 210 is equipped with a display unit for displaying a surgical environment of the surgical instrument, and also equipped with a surgeon operation control mechanism, an armrest, etc.. The display unit includes an observation window for the surgeon to observe. The operation control mechanism is used to be manipulated so that the manipulation of the operation control mechanism effects movement of the respective surgical instrument. The armrest is used for the surgeon's arms to rest on. In addition, the surgeon console 210 further includes other control switches that are convenient for hands or feet to touch or pressing. The control switches are used for various functional operations to complete human-computer interaction.

The imaging system 230 includes a display screen, an endoscope controller, system electronic devices, and an image processor.

Referring to FIG. 2, the robotic arm system 220 may include at least one robotic arm 221. The robotic arm 221 includes multiple links among which two adjacent links may move relative to each other with a specific degree of freedom, resulting in multiple degrees of freedom of motions for the end of the robotic arm (such as seven degrees of freedom, which may vary depending on the surgical instrument). The end of the robotic arm 221 is equipped with an instrument holder 222. The surgical instrument 100 is removably mounted on the instrument holder 222. The surgical instrument 100 may be an instrument used for surgical operations, such as an electrocautery instrument, a clamp, or a vessel sealer. The surgical instrument 100 may also be a camera for capturing images in the surgical area, such as an endoscopic camera. The surgical instrument 100 may also be other surgical instruments.

In some embodiments, the robotic arm 221 may be designed to move around a remote center of motion (RCM) maintained mechanically. For instance, during a laparoscopic surgery, the RCM is aligned with the incision point to the patient's abdominal cavity. During the surgery, the robotic arm 221 is manipulated to move the instrument holder 222, thereby moving the surgical instrument 100 to perform motions such as pitch, yaw, insertion, and roll. During the above motions, the longitudinal axis of the surgical instrument 100 is constrained to the RCM, thereby preventing any non-surgical damage to the patient's abdominal incision caused by the surgical instrument 110.

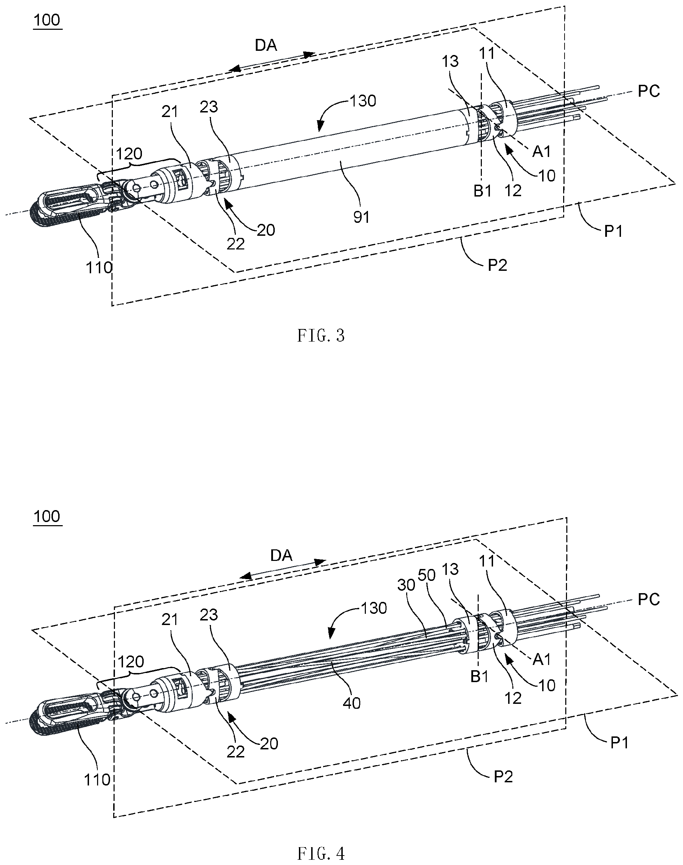

The surgical instrument 100 sequentially includes, from the proximal end to the distal end, a rear-end transmission device 150, a shaft 140, and an end effector 110. The rear-end transmission device 150 is engaged with a driving device on the instrument holder 222. The rear-end transmission device 150 may be coupled to the end effector 110 through transmission members such as push-pull rods, wires, ropes, and belts. The shaft 140 is coupled to and disposed between the rear-end transmission device 150 and the end effector 110 for maintenance of a certain distance of the rear-end transmission device 150 from the end effector 110 and for support of the end effector 110. The end effector 110 may be a tool, such as a hook, a spatula, a needle, a clamp, or a pair of scissors, for performing surgical operations such as tissue cutting, etc.. The end effector 110 may also be an endoscope lens for capturing images. For example, in the embodiments of the present disclosure, the end effector 110 is a clamp as shown in FIG. 4, which includes a first jaw 111 and a second jaw 112 capable of performing opening and closing motions.

Furthermore, joints such as a wrist, a parallel motion mechanism, and/or an elbow may further be employed to couple the end effector 110 to the shaft 140, thereby enhancing the mobility of the end effector 110. The rear-end driving device 150 may acuate the joints through transmission members such as push-pull rods, wires, ropes, and belts.

Referring to FIGS. 3 and 4, the surgical instrument 100 of the embodiment of the present disclosure includes a wrist 120 and a parallel motion mechanism 130. The end effector 110 is coupled to the distal end of the wrist 120, and the proximal end of the wrist 120 is coupled to the distal end of the parallel motion mechanism 130. In the embodiments of the present disclosure, the wrist 120 is closer to the end effector 110 compared to other joints, and therefore also referred to as an end joint assembly in the present disclosure. The end joint assembly 120 may include a pitch joint and/or a yaw joint. A rear-end driving device (not shown) stops the end joint assembly 120 through at least one pair of end joint driving wires 50. The quantity of the end joint driving wires 50 is related to the degrees of freedom of motions of the end joint assembly 120.

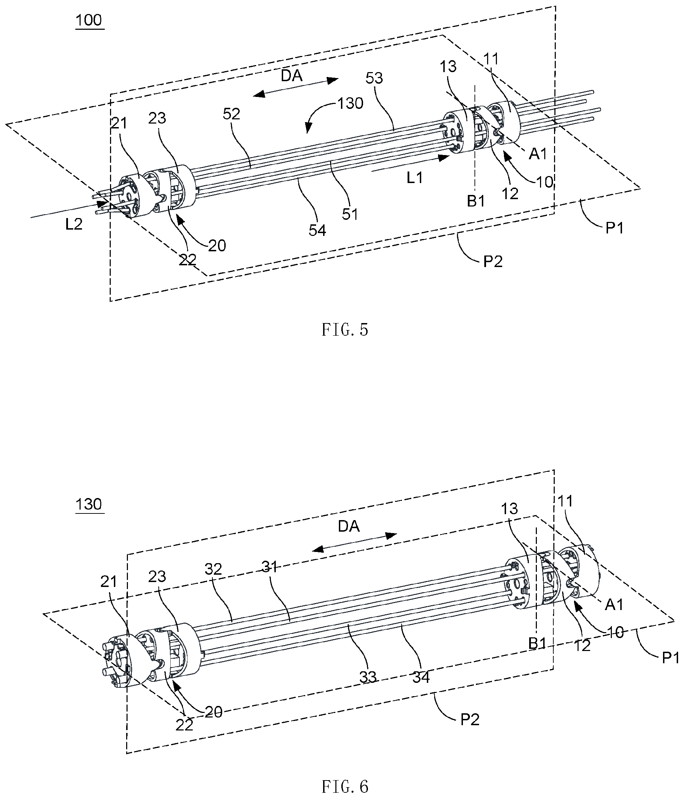

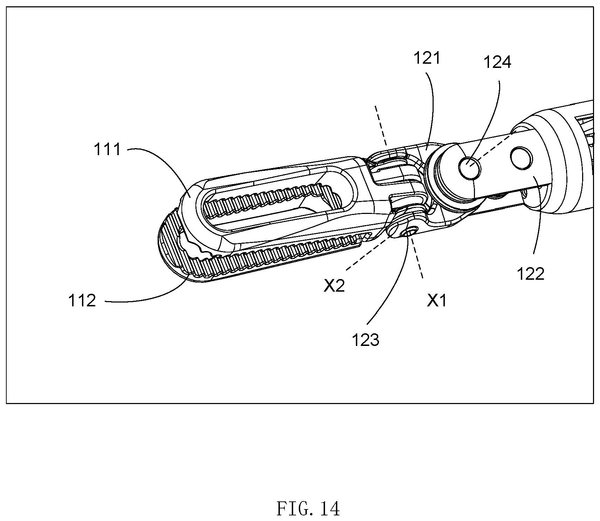

For example, in the embodiments of the present disclosure, the end joint assembly 120 includes a pitch joint and a yaw joint. Specifically, referring to FIG. 14, the end joint assembly 120 includes a yaw joint base 121 and a pitch joint base 122. The end effector 110 has a proximal end coupled to the distal end of the yaw joint base 121 and rotatable about a yaw axis X1 relative to the yaw joint base 121, thereby forming the yaw joint. The yaw joint base 121 has a proximal end coupled to the distal end of the pitch joint base 122 and rotatable about a pitch axis X2 relative to the pitch joint base 122, thereby forming the pitch joint. The yaw axis X1 and the pitch axis X2 are non-coplanar, and are optionally perpendicular to each other and non-coplanar. For example, the yaw joint base 121 may be designed to be U-shaped. The proximal ends of the first jaw 111 and the second jaw 112 of the end effector 110 are located in the U-shaped space of the yaw joint base 121, and pivotably coupled to the yaw joint base 121 through a first clevis pin 123. The yaw motion of the end joint assembly 120 and the opening and closing motion of the end effector 110 are achieved through the rotation of the first jaw 111 and the second jaw 112. For example, the pitch joint base 122 may also be designed to be U-shaped. The proximal end of the yaw joint base 121 are located in the U-shaped space of the pitch joint base 122, and pivotably coupled to the pitch joint base 122 through a second clevis pin 124. The pitch motion of the end joint assembly 120 is achieved through the rotation of the yaw joint base 121. The proximal end of the pitch joint base 122 is coupled to the distal end of the parallel motion mechanism 130, such that the end joint assembly 120 and the end effector 110 are movable together with the distal end of the parallel motion mechanism 130. Since the end joint assembly 120 in the embodiment has two degrees of freedom including pitch and yaw, at least two pairs of end joint driving wires 50 are required as shown in FIG. 5 (denoted as 51, 52, 53, 54 in FIG. 5). In the embodiments of the present disclosure, each of the pitch joint and the yaw joint of the end joint assembly 120 is a pivot joint. However, it is understood that the pitch joint and the yaw joint may also be designed as rolling joints, a combination of a pivot joint and a rolling joint, or serpentine joints.

It is understandable that in other embodiments not shown, as a variation of the embodiment in the present disclosure, the wrist 120 may also be omitted between the end effector 110 and the parallel motion mechanism 130.

The parallel motion mechanism 130 according to the embodiment of the present disclosure has at least two degrees of freedom of motions.

Referring to FIGS. 3 to 9, the parallel motion mechanism 130 includes a proximal joint assembly 10 and a distal joint 20 assembly. The proximal joint assembly 10 is disposed at the proximal end of the parallel motion mechanism 130, and used to couple to a shaft (not shown in the above figures). The distal joint assembly 20 is disposed at the distal end of the parallel motion mechanism 130. The distal joint assembly 20 is used to couple to the end effector 110.

The proximal joint assembly 10 includes a first proximal joint portion 11, a second proximal joint portion 12, and a third proximal joint portion 13, which are coupled in series. The first proximal joint portion 11 is disposed at the proximal end of the parallel motion mechanism 130. The second proximal joint portion 12 is coupled to the distal end of the first proximal joint portion 11, and can swing relative to the first proximal joint portion 11 along a second plane P2. That is, the motion trajectory of any point of the second proximal joint portion 12 relative to the first proximal joint portion 11 is constrained within the second plane P2 or a plane parallel to the second plane P2. The third proximal joint portion 13 is coupled to the distal end of the second proximal joint portion 12, and can swing relative to the second proximal joint portion 12 along a first plane P1. That is, the motion trajectory of any point of the third proximal joint portion 13 relative to the second proximal joint portion 12 is constrained within the first plane P1 or a plane parallel to the first plane P1.

The first plane P1 and the second plane P2 are two planes each passing through the central axis PC of the parallel motion mechanism 130. That is, the first plane P1 intersects with the second plane P2 at the central axis PC of the parallel motion mechanism 130. Optionally, the first plane P1 is perpendicular to the second plane P2. The central axis PC of the parallel motion mechanism 130 refers to the central axis when the parallel motion mechanism 130 is in a zero-position state (also known as a neutral state).

The distal joint assembly 20 includes a first distal joint portion 21, a second distal joint portion 22, and a third distal joint portion 23, which are coupled in series. The third distal joint portion 23 and the third proximal joint portion 13 are coupled to each other and fixed relative to each other, such that the relative position and orientation between them remain unchanged. The second distal joint portion 22 is coupled to the distal end of the third distal joint portion 23, and can swing relative to the third distal joint portion 23 along the first plane P1 mentioned above. That is, the motion trajectory of any point of the second distal joint portion 22 relative to the third distal joint portion 23 is constrained within the first plane P1 or a plane parallel to the first plane P1. The first distal joint portion 21 is coupled to the distal end of the second distal joint portion 22, and can swing relative to the second distal joint portion 22 along the second plane P2 mentioned above. That is, the motion trajectory of any point of the first distal joint portion 21 relative to the second distal joint portion 22 is constrained within the second plane P2 or a plane parallel to the second plane P2.

Optionally, in the embodiment of the present disclosure, the third distal joint portion 23 is coupled to the third proximal joint portion 13 through a sleeve 91. The sleeve 91 may be rigid that can maintain its shape, so that the relative position and orientation between the third distal joint portion 23 and the third proximal joint portion 13 remain unchanged. The sleeve 91 is designed as, for example, an elongated cylindrical tube for increasing the radius of motion area of the parallel motion mechanism 130 on the one hand, and allowing the wire to pass through on the other hand. The axial direction DA of the sleeve 91 is also the axial direction of the parallel motion mechanism 130. The central axis of the sleeve 91 coincides with the central axis PC of the parallel motion mechanism 130. In other embodiments not shown, the proximal end of the third distal joint portion 23 and the distal end of the third proximal joint portion 13 may be directly coupled to each other such as by threads, an adhesive, an interlock engagement, or other manners for examples.

As shown in FIGS. 4 and 6, to maintain a same orientation of the first distal joint portion 21 and the first proximal joint portion 11 to achieve parallel motion, the parallel motion mechanism 130 further includes a plurality of constraint wires 30. Each constraint wire 30 has an end fixed to the first proximal joint portion 11 and another end fixed to the first distal joint portion 21. For example, in this embodiment, one end of each constraint wire 30 is disposed at the first proximal joint portion 11, such as being stuck at the surface of the proximal end of the first proximal joint portion 11 with a wiring terminal. Then the constraint wire 30 is sequentially routed through the second proximal joint portion 12, the third proximal joint portion 13, the sleeve 91, the third distal joint portion 23, and the second distal joint portion 22, with its another end being disposed at the first distal joint portion 21, such as being stuck at the surface of the distal end of the first distal joint portion 21 with another wiring terminal. Each constraint wire 30 may be made of a rigid material, and may be a steel wire or a tungsten wire for example. It can be understood that each constraint wire 30 is kept being tensioned within the parallel motion mechanism 130, so that the length of the constraint wire 30 within the parallel motion mechanism 130 remains constant. The constraint wires 30 are routed such that the proximal joint assembly 10 and the distal joint assembly 20 swing at equal angles and in opposite directions, and such that the two degrees of freedom of motions of the parallel motion mechanism 130 are independent of each other.

As shown in FIG. 4, to actuate the parallel motion mechanism 130 for parallel motion, the parallel motion mechanism 130 further includes a plurality of driving wires 40. In the embodiment of the present disclosure, the driving wires 40 are configured to actuate the distal joint assembly 20, and the movement of the distal joint assembly 20 stops the proximal joint assembly 10 via the constraint wires 30. For example, in this embodiment, each driving wire 40 has one end fixed to the first distal joint portion 21, for example, stuck at the surface of the distal end of the first distal joint portion 21 with a wiring terminal. The driving wire 40 is then sequentially routed through the second distal joint portion 22, the third distal joint portion 23, the sleeve 91, the third proximal joint portion 13, the second proximal joint portion 12, and the first proximal joint portion 11, and with its another end coupled to the rear-end transmission device located at the proximal end of the surgical instrument.

As shown in FIGS. 3 to 7, when the parallel motion mechanism 130 has no deflection, that is, when parallel motion mechanism 130 is in the zero-position state (also known as the neutral state), the first proximal joint portion 11, the second proximal joint portion 12, the third proximal joint portion 13, the third distal joint portion 23, the second distal joint portion 22, and the first distal joint portion 21 are sequentially arranged along the central axis PC and aligned with each other, so that the parallel motion mechanism 130 appears as a linear structure.

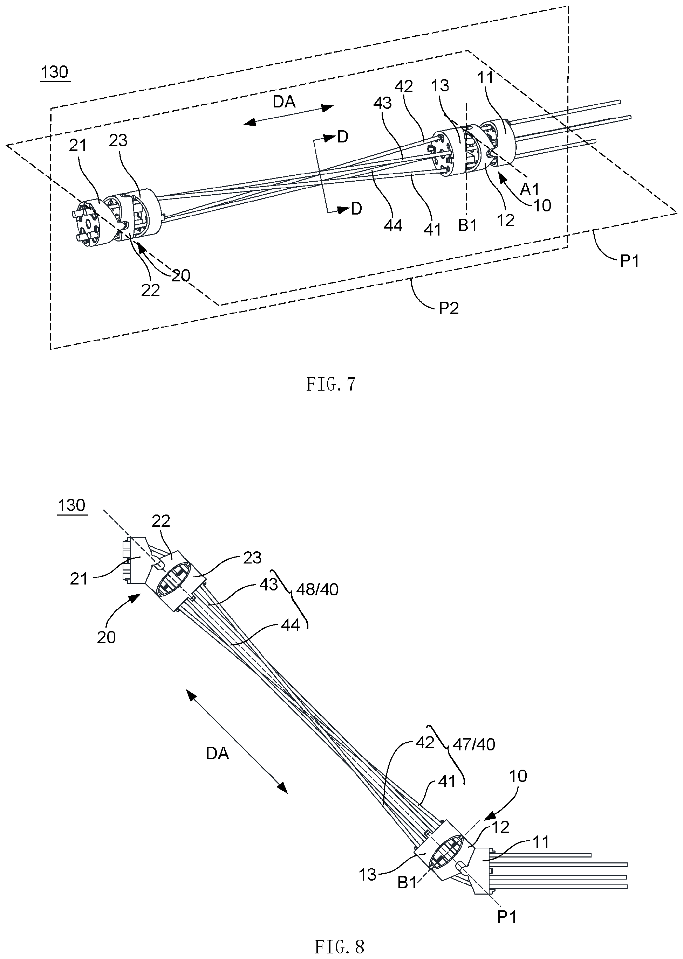

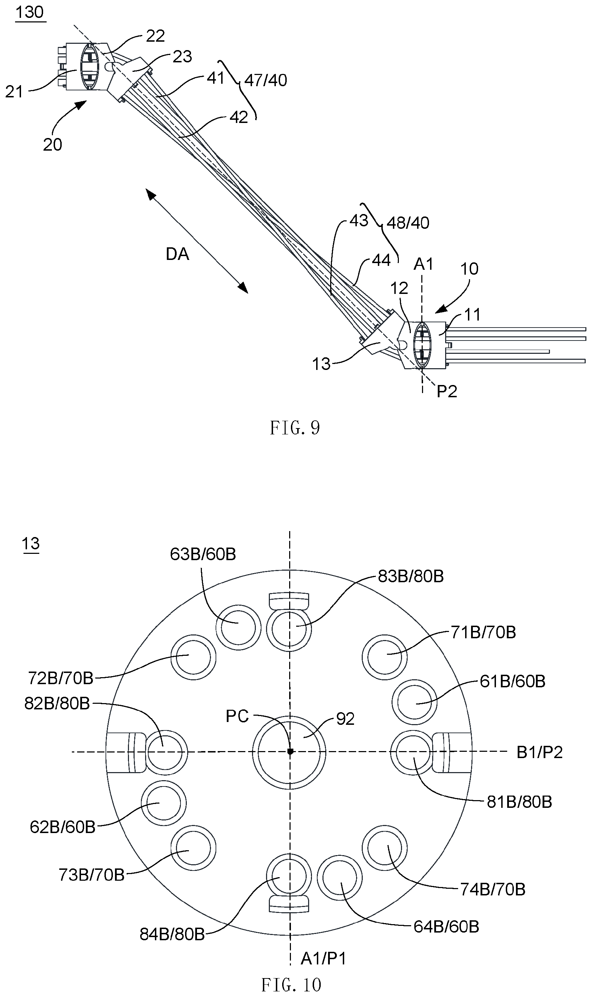

As shown in FIGS. 8 and 9, when the rear-end transmission device outputs driving force, the driving wires 40 transmit the driving force to the distal joint assembly 20, thereby rotating the first distal joint portion 21 relative to the second distal joint portion 22 (see FIG. 8), and/or rotating the second distal joint portion 22 relative to the third distal joint portion 23 (see FIG. 9). At this time, the parallel motion mechanism 130 exhibits a shape in which two ends are bent in opposite directions relative to the middle portion, which is referred to as a deflection state of the parallel motion mechanism 130.

Optionally, in the embodiment of the present disclosure, the parallel motion mechanism 130 includes two pairs of constraint wires 30 as shown in FIG. 6 (denoted as 31, 32, 33, 34 in FIG. 6). When the parallel motion mechanism 130 is in the neutral state, the two pairs of constraint wires 30 are symmetrically arranged about both of the first plane P1 and the second plane P2, and are optionally routed parallel to the central axis PC. Since the length of each constraint wire 30 remains unchanged, the above arrangement of the constraint wires allows that a change in the length of each constraint wire 30 routed through the distal joint assembly 20 results in an opposite change in the length of the constraint wire 30 routed through the proximal joint assembly 10, thereby achieving parallel motion of the parallel motion mechanism 130 while the two degrees of freedom of motions of the parallel motion mechanism 130 are independent of each other.

Specifically, when the first distal joint portion 21 is rotated by a first angle relative to the second distal joint portion 22, one side of the first distal joint portion 21 moves away from the second distal joint portion 22, while the other side of the first distal joint portion 21 moves toward the second distal joint portion 22 (as shown in FIG. 8). For example, the length of each of the constraint wires 31 and 32 routed between the first distal joint portion 21 and the second distal joint portion 22 increases by a first length, while the length of each of the constraint wires 31 and 32 routed between the second proximal joint portion 12 and the first proximal joint portion 11 decreases by the first length accordingly. At the same time, the length of each of the constraint wires 33 and 34 routed between the first distal joint portion 21 and the second distal joint portion 22 decreases by the first length, while the length of each of the constraint wires 33 and 34 routed between the second proximal joint portion 12 and the first proximal joint portion 11 increases by the first length accordingly. Thereby, the second proximal joint portion 12 is meanwhile rotated by the first angle relative to the first proximal joint portion 11, and the rotation direction is opposite to that of the first distal joint portion 21 relative to the second distal joint portion 22.

When the second distal joint portion 22 is rotated by a second angle relative to the third distal joint portion 23, one side of the second distal joint portion 22 moves away from the third distal joint portion 23, while the other side of the second distal joint portion 22 moves toward the third distal joint portion 23 (as shown in FIG. 9). For example, the length of each of the constraint wires 31 and 34 routed between the second distal joint portion 22 and the third distal joint portion 23 increases by a second length, while the length of each of the constraint wires 31 and 34 routed between the third proximal joint portion 13 and the second proximal joint portion 12 decreases by the second length accordingly. At the same time, the length of each of the constraint wires 32 and 33 routed between the second distal joint portion 22 and the third distal joint portion 23 decreases by the second length, while the length of each of the constraint wires 32 and 33 routed between the third proximal joint portion 13 and the second proximal joint portion 12 increases by the second length accordingly. Thereby, the third proximal joint portion 13 is meanwhile rotated by the second angle relative to the second proximal joint portion 12, and the rotation direction is opposite to that of the second distal joint portion 22 relative to the third distal joint portion 23.

Thus, during defection of the parallel motion mechanism 130, the first proximal joint portion 11 and the first distal joint portion 21 are always parallel to each other (i.e., have a same orientation), such that the end effector 110 is moved without changing its orientation. In the present disclosure, the end effector 110 can be moved in two degrees of freedom.

In the embodiment of the present disclosure, since the driving wires 40 are routed through the entire parallel motion mechanism 130 that is movable in two degrees of freedom, it is important to ensure that the two degrees of freedom of motions of the parallel motion mechanism 130 are independent of each other. That is, it is needed to decouple the driving wires 40 in the two degrees of freedom of motions of the parallel motion mechanism 130. For this purpose, in the embodiment of the present disclosure, the driving wires 40 is configured such that during the movement of the parallel motion mechanism 130, a sum of lengths of the driving wires 40 routed through the parallel motion mechanism 130 remains unchanged.

In the embodiment of the present disclosure, two pairs of driving wires 40 are included to control the two degrees of freedom of motions, respectively. During the movement of the parallel motion mechanism 130, the sum of lengths of each pair of the driving wires 40 routed through the parallel motion mechanism 130 remains unchanged. The above configuration of the driving wires 40 allows the driving wires 40 to cooperate with an existing rear-end drive mechanism to stop the parallel motion mechanism 130, and to decouple the two degrees of freedom of motions.

Specifically, as shown in FIGS. 7 to 9, the driving wires 40 include a first pair of driving wires 47 and a second pair of driving wires 48. The first pair of driving wires 47 includes a first driving wire 41 and a second driving wire 42, which are used to drive the first distal joint portion 21 to swing relative to the second distal joint portion 22 along the second plane P2. The second pair of driving wires 48 includes a third driving wire 43 and a fourth driving wire 44, which are used to drive the second distal joint portion 22 to rotate relative to the third distal joint portion 23.

Correspondingly, the rear-end transmission device includes a first transmission device and a second transmission device. The first transmission device is coupled to the first pair of driving wires 47 for changing the lengths of the first driving wire 41 and the second driving wire 42. The second transmission device is coupled to the second pair of driving wires 48 for changing the lengths of the third driving wire 43 and the fourth driving wire 44. Each of the first driving wire 41 and the second driving wire 42 has one end coupled to the first transmission device, and then is routed through the shaft, the first proximal joint portion 11, the second proximal joint portion 12, the third proximal joint portion 13, the third distal joint portion 23, and the second distal joint portion 22, and has the other end coupled to the first distal joint portion 21. Each of the third driving wire 43 and the fourth driving wire 44 has one end coupled to the second transmission device, and then is routed through the shaft, the first proximal joint portion 11, the second proximal joint portion 12, the third proximal joint portion 13, the third distal joint portion 23, and the second distal joint portion 22, and has the other end coupled to the first distal joint portion 21.

Since the sum of lengths of the first pair of driving wires 47 routed through the parallel motion mechanism 130 remains unchanged and the length of the shaft is constant, the sum of lengths of the first driving wire 41 and the second driving wire 42 may be designed to be unchanged. For example, the first transmission device may be designed as a first capstan, and the first driving wire 41 and the second driving wire 42 are wound in opposite directions on the first capstan. When the parallel motion mechanism 130 is in the neutral state, the length of the first driving wire 41 is equivalent to the length of the second driving wire 42. When the first capstan is rotated, the length of the first driving wire 41 being pulled (or retracted) is equal to the length of the second driving wire 42 being retracted (or pulled), such that the sum of lengths of the first driving wire 41 and the second driving wire 42 remains unchanged. Similarly, the sum of lengths of the third driving wire 43 and the fourth driving wire 44 remains unchanged. For example, the second transmission device is designed as a second capstan, and the third driving wire 43 and the fourth driving wire 44 are wound in opposite directions on the second capstan. For ease of understanding, an exemplary arrangement of the driving wires 40 will be described in detail later with reference to the arrangement of wire through holes.

As mentioned earlier, the surgical instrument 100 in the embodiment may further include the end joint assembly 120 and at least one pair of end joint driving wires 50.

As shown in FIGS. 4 and 5, the end joint assembly 120 is disposed between the first distal joint portion 21 and the end effector 110, which moves the end effector 110 relative to the distal joint assembly 20. The end joint driving wires 50 are used to actuate the end joint assembly 120. Each end driving wire 50 has one end coupled to the rear-end transmission device and then is routed through the shaft and the parallel motion mechanism 130, and has another end coupled to the end joint assembly 120. To prevent the motions of the parallel motion mechanism 130 from affecting the control of the end joint assembly 120 by the end driving wires 50, it is necessary to decouple the motion of the end driving wires 50 and the motion of the parallel motion mechanism 130. Thus, the end driving wires 50 may be configured such that when the parallel motion mechanism 130 is in the neutral state, the end driving wires 50 are routed at the first plane P1 and/or the second plane P2 and parallel to the central axis PC. The above configuration ensures that when the parallel motion mechanism 130 moves, the increase (decrease) amount in the length of each end driving wire 50 routed through the proximal joint assembly 10 is equal to the decrease (increase) amount in the length of the respective end joint driving wire 50 routed through the distal joint assembly 20. That is, the length of each end driving wire 50 routed through the parallel motion mechanism 130 remains unchanged, such that the motion of the parallel motion mechanism 130 will not result in any change in the length of the end driving wire 50. Thus, decoupling of the motion of the end driving wires 50 and the motion of the parallel motion mechanism 130 is achieved.

The quantity of the end driving wires 50 may be set according to the degrees of freedom of the end joint assembly 120. For example, in the embodiment of the present disclosure, the end joint assembly 120 includes two degrees of freedom including pitch and yaw. Therefore, at least two pairs of end driving wires 50 are required to control the motions of the end joint assembly 120. One pair of end driving wires 50 controls the pitch motion of the end joint assembly 120, and another pair of end driving wires 50 controls the yaw motion of the end joint assembly 120. In a case that the end effector 110 is a tool such as a clamp or a pair of scissors for performing opening and closing motion, the opening and closing motion of the end effector 110 may be actuated by the pair of end driving wires 50 that also actuates the yaw motion, and the specific control method and the specific structure and working principle of the rear-end transmission device 150 that cooperates with the end joint driving wires 50 can refer to existing solutions, such as those disclosed in Chinese patent applications CN113208732A or CN113367796A, which will not be repeated here. Alternatively, the opening and closing motion of the end effector 110 may also be actuate by additional end driving wires. In the embodiment of the present disclosure, the surgical instrument 100 includes four end joint driving wires 50, namely a first end joint driving wire 51, a second end joint driving wire 52, a third end joint driving wire 53, and a fourth end joint driving wire 54. When the parallel motion mechanism 130 is in the neutral state, the four end joint driving wires 50 are routed parallelly through the parallel motion mechanism 130. Optionally, the four end joint driving wires 50 may be symmetrically arranged about both of the first plane P1 and the second plane P2, which is beneficial for simplifying the control of the end joint driving wires 50.

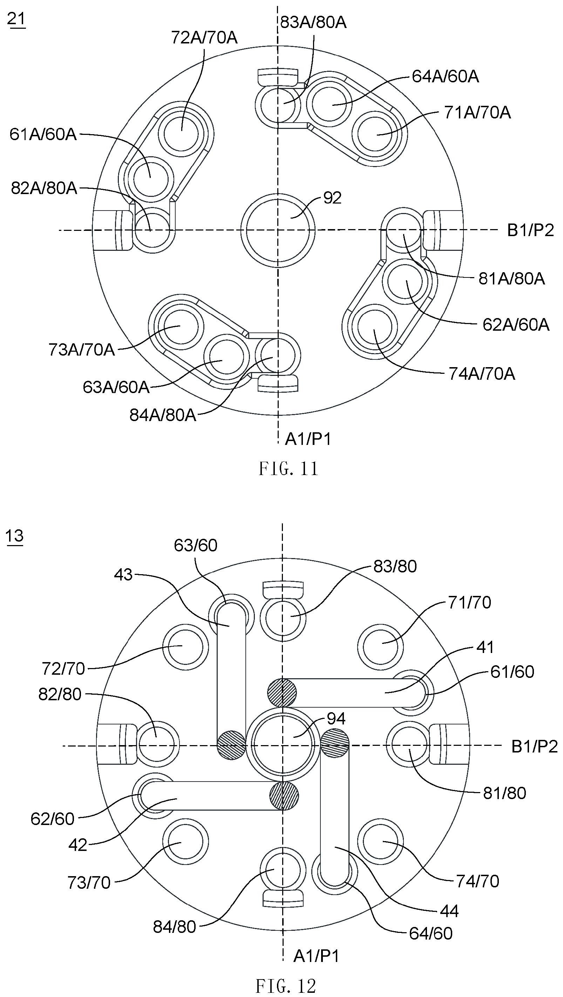

As mentioned above, in the embodiment of the present disclosure, the surgical instrument 100 includes four constraint wires 30, four driving wires 40 (also referred to as parallel motion driving wires 40), and four end joint driving wires 50. Each of the twelve wires is sequentially routed through the first proximal joint portion 11, the second proximal joint portion 12, the third proximal joint portion 13, the third distal joint portion 23, the second distal joint portion 22, and the first distal joint portion 21. Therefore, as shown in FIGS. 10 and 11, each of the first proximal joint portion 11, the second proximal joint portion 12, and the third proximal joint portion 13 defines four proximal through holes 70B for the constraint wires 30 to pass through, four proximal through holes 60B for the parallel motion driving wires 40 to pass through, and four proximal through holes 80B for the end joint driving wires 50 to pass through. Each of the third distal joint portion 23, the second distal joint portion 22, and the first distal joint portion 21 defines four distal through holes 70A for the constraint wires 30 to pass through, four distal through holes 60A for the parallel motion driving wires 40 to pass through, and four distal through holes 80A for the end joint driving wires 50 to pass through.

Each parallel motion driving wire 40 has a section routed through the proximal joint assembly 10 and a section routed through the distal joint assembly 20. Optionally, both of the sections are routed parallel to the central axis PC in the neutral state. In addition, as mentioned above, each of the constraint wires 30 and the end joint driving wires 50 is routed parallel to the central axis PC in the neutral state. Therefore, the wire through holes in the first proximal joint portion 11, the second proximal joint portion 12, and the third proximal joint portion 13 (including three wire through holes in the embodiment of the present disclosure) for one wire to pass through are aligned with each other in a direction parallel to the central axis PC. The wire through holes in the third distal joint portion 23, the second distal joint portion 22, and the first distal joint portion 21 for one wire to pass through are aligned with each other in a direction parallel to the central axis PC.

The following will describe the arrangement of the twelve wires, with reference to the twelve wire through holes of the third proximal joint portion 13 (see FIG. 10) and the twelve wire through holes of the first distal joint portion 21 (see FIG. 11). FIGS. 10 and 11 show the third proximal joint portion 13 and the first distal joint portion 21, respectively, when the parallel motion mechanism 130 is in the neutral state, where each of the first plane P1, the second plane P2, and the central axis PC is perpendicular to the paper surface.

For the four end joint driving wires 50, the proximal joint assembly 10 defines four sets of proximal through holes 80B for end joint driving wires. Taking the third proximal joint portion 13 as an example, referring to FIG. 10, there are a first proximal driving wire through hole 81B, a second proximal driving wire through hole 82B, a third proximal driving wire through hole 83B, and a fourth proximal driving wire through hole 84B. The distal joint assembly 20 defines four sets of distal through holes 80A for end joint driving wires. Taking the first distal joint portion 21 as an example, referring to FIG. 11, there are a first distal driving wire through hole 81A, a second distal driving wire through hole 82A, a third distal driving wire through hole 83A, and a fourth distal driving wire through hole 84A. The first proximal driving wire through hole 81B and the first distal driving wire through hole 81A are used for routing the first end joint driving wire 51. The second proximal driving wire through hole 82B and the second distal driving wire through hole 82A are for routing the second end joint driving wire 52. The third proximal driving wire through hole 83B and the third distal driving wire through hole 83A are used for routing the third end joint driving wire 53. The fourth proximal driving wire through hole 84B and the fourth distal driving wire through hole 84A are used for routing the fourth end joint driving wire 54.

As shown in FIG. 10, the through holes 81B, 82B, 83B, and 84B are arranged rotationally symmetrically about the central axis PC. In the neutral state, the through holes 81B and 82B are arranged symmetrically with respect to the first plane P1, and the centers of the through holes 81B and 82B are located on the second plane P2. Thus, sections of the first end joint driving wire 51 and the second end joint driving wire 52, which are routed through the proximal joint assembly 10, are arranged symmetrically with respect to the first plane P1 and routed at the second plane P2. The through holes 83B and 84B are arranged symmetrically with respect to the second plane P2, and the centers of the through holes 83B and 84B are located on the first plane P1. Thus, sections of the third end joint driving wire 53 and the fourth end joint driving wire 54, which are routed through the proximal joint assembly 10, are arranged symmetrically with respect to the second plane P2 and routed at the first plane P1. As shown in FIG. 11, the arrangement of the through holes 81A, 82A, 83A, and 84A in the distal joint assembly 20 is similar to that in the proximal joint assembly 10 in FIG. 10, which will not be repeated in the description.

As shown in FIGS. 10 and 11, the through holes 81B, 82B, 83B, and 84B of the proximal joint assembly 10 are aligned with the through holes 81A, 82A, 83A, and 84A of the distal joint assembly 20, respectively, in a direction parallel to the central axis PC. Therefore, in the neutral state, the four end joint driving wires 51, 52, 53, and 54 are parallel to the central axis PC.

For the four constraint wires 30, the proximal joint assembly 10 defines four sets of proximal through holes 70B for end constraint wires. Taking the third proximal joint portion 13 as an example, referring to FIG. 10, there are a first proximal constraint wire through hole 71B, a second proximal constraint wire through hole 72B, a third proximal constraint wire through hole 73B, and a fourth proximal constraint wire through hole 74B. The distal joint assembly 20 defines four sets of distal through holes 70A for end constraint wires. Taking the first distal joint portion 21 as an example, referring to FIG. 11, there are a first distal constraint wire through hole 71A, a second distal constraint wire through hole 72A, a third distal constraint wire through hole 73A, and a fourth distal constraint wire through hole 74A. The first proximal constraint wire through hole 71B and the first distal constraint wire through hole 71A are used for routing the first constraint wire 31. The second proximal constraint wire through hole 72B and the second distal constraint wire through hole 72A are used for routing the second constraint wire 32. The third proximal constraint wire through hole 73B and the third distal constraint wire through hole 73A are used for routing the third constraint wire 33. The fourth proximal constraint wire through hole 74B and the fourth distal constraint wire through hole 74A are used for routing the fourth constraint wire 34.

As shown in FIG. 10, the through holes 71B, 72B, 73B, and 74B are arranged rotationally symmetrically about the central axis PC. The through holes 71B, 72B, 73B, and 74B are arranged symmetrically with respect to each of the first plane P1 and the second plane P2. Thus, sections of the four constraint wires 30, which are routed through the proximal joint assembly 10, are arranged symmetrically with respect to each of the first plane P1 and the second plane P2. Furthermore, each of the through holes 71B, 72B, 73B, and 74B is equidistant from to the first plane P1 and the second plane P2. That is, a line connecting the center of each of the through holes 71B, 72B, 73B, and 74B to the central axis PC bisects the angle defined by the first plane P1 and the second plane P2. As shown in FIG. 11, the arrangement of the through holes 71B, 72B, 73B, and 74B in the distal joint assembly 20 is similar to that in the proximal joint assembly 10 in FIG. 10, which will not be repeated in the description.

Furthermore, as shown in FIGS. 10 and 11, the through holes 71B, 72B, 73B, and 74B of the proximal joint assembly 10 are aligned with the through holes 71A, 72A, 73A, and 74A of the distal joint assembly 20 in a direction parallel to the central axis PC. Therefore, in the neutral state, each of the four constraint wires 31, 32, 33, and 34 are parallel to the central axis PC.

For the four parallel motion driving wires 40, the proximal joint assembly 10 defines four sets of proximal through holes 60B for parallel motion driving wires. Taking the third proximal joint portion 13 as an example, referring to FIG. 10, there are a first proximal through hole 61B, a second proximal through hole 62B, a third proximal through hole 63B, and a fourth proximal through hole 64B. The distal joint assembly 20 defines four sets of distal through holes 60A for parallel motion driving wires. Taking the first distal joint portion 21 as an example, referring to FIG. 11, there are a first distal through hole 61A, a second distal through hole 62A, a third distal through hole 63A, and a fourth distal through hole 64A. The first proximal through hole 61B and the first distal through hole 61A are used for routing the first driving wire 41. The second proximal through hole 62B and the second distal through hole 62A are used for routing the second driving wire 42. The third proximal through hole 63B and the third distal through hole 63A are used for routing the third driving wire 43. The fourth proximal through hole 64B and the fourth distal through hole 64A are used for routing the fourth driving wire 44.

Unlike the through holes 70B, 70A and the through holes 80B, 80A, the proximal through holes 60B for parallel motion driving wires are not aligned with the distal through holes 60A for parallel motion driving wires in a direction parallel to the central axis PC. Thus, the sections of the four driving wires 40 routed between the third distal joint portion 23 and the third proximal joint portion 13 are not parallel to the central axis PC.

Specifically, the first proximal through hole 61B and the first distal through hole 61A are located at two opposite sides of the first plane P1, and are equidistant from the first plane P1. Similarly, the second proximal through hole 62B and the second distal through hole 62A are located at two opposite sides of the first plane P1, and are equidistant from the first plane P1. Thus, the section of each of the driving wires 41 and 42 of the first pair of driving wires 47 routed through the proximal joint assembly 10 and the section of the same driving wire routed through the distal joint assembly 20 are located on two opposite sides of the first plane P1, and are equidistant from the first plane P1. The above arrangement ensures that when the parallel motion mechanism 130 swings along the first plane P1, the increase (decrease) amount in length of each of the driving wire 41 and 42 of the first pair of driving wires 47 routed through the proximal joint assembly 10 is equal to the decrease (increase) amount in length of the same driving wire routed through the distal joint assembly 20. Therefore, the first pair of driving wires 47, which controls the parallel motion mechanism 130 to swing along the second plane P2, is decoupled from the swing of the parallel motion mechanism 130 along the first plane P1. That is, the swing of the parallel motion mechanism 130 along the first plane P1 causes no change in the length of the first pair of driving wires 47.

Optionally, the first proximal through hole 61B and the second proximal through hole 62B are located at two opposite sides of the first plane P1, and thus the first distal through hole 61A and the second distal through hole 62A are located at two opposite sides of the first plane P1. Thus, the section of the first driving wire 41 routed through the proximal joint assembly 10 and the section of the second driving wire 42 routed through the proximal joint assembly 10 are located at two opposite sides of the first plane P1, and the section of the first driving wire 41 routed through the distal joint assembly 20 and the section of the second driving wire 42 routed through the distal joint assembly 20 are located at two opposite sides of the first plane P1. The above arrangement allows the first driving wire 41 and the second driving wire 42 to independently control the parallel motion mechanism 130 to swing along the second plane P2 in opposite directions, respectively.

Furthermore, optionally, the distance from the first proximal through hole 61B to the first plane P1 is equal to the distance from the second proximal through hole 62B to the first plane P1, and thus the distance from the first distal through hole 61A to the first plane P1 is equal to the distance from the second distal through hole 62A to the first plane P1. Thus, the distance from the section of the first driving wire 41 routed through the proximal joint assembly 10 to the first plane P1 is equal to the distance from the section of the second driving wire 42 routed through the proximal joint assembly 10 to the first plane P1, and the distance from the section of the first driving wire 41 routed through the distal joint assembly 20 to the first plane P1 is equal to the distance from the section of the second driving wire 42 routed through the distal joint assembly 20 to the first plane P1. The above arrangement allows the driving forces for the first driving wire 41 and the second driving wire 42 to be equal.

Furthermore, optionally, the first proximal through hole 61B and the second proximal through hole 62B are arranged with 180-degree rotational symmetry about the central axis PC. The first distal through hole 61A and the second distal through hole 62A are arranged with 180-degree rotational symmetry about the central axis PC. The first proximal through hole 61B and the first distal through hole 61A are located at a same side of the second plane P2, and are equidistant from the second plane P2. The second proximal through hole 62B and the second distal through hole 62A are located at a same side of the second plane P2, and are equidistant from the second plane P2. The first proximal through hole 61 and the second proximal through hole 62 are located at two opposite sides of the second plane P2, respectively. The first distal through hole 61A and the second distal through hole 62A are located at two opposite sides of the second plane P2, respectively. The first driving wire 41 and the second driving wire 42 are routed at two opposite sides of the second plane P2, respectively. As shown in FIGS. 7, 8, and 12, furthermore, each of the first driving wire 41 and the second driving wire 42 is routed parallel to the second plane P2, and the route of each of the first driving wire 41 and the second driving wire 42 between the third distal joint portion 23 and the third proximal joint portion 13 is straight. Therefore, the first driving wire 41 and the second driving wire 42, which are routed between the third distal joint portion 23 and the third proximal joint portion 13, are arranged with 180-degree rotational symmetry about the central axis PC. The above arrangement allows the lengths of the first driving wire 41 and the second driving wire 42 to be equal when the parallel motion mechanism 130 is in the neutral state, which may simplify the rear-end transmission device.

Similarly, the third proximal through hole 63B and the third distal through hole 63A are located at two opposite sides of the second plane P2, respectively, and equidistant from the second plane P2. Similarly, the fourth proximal through hole 64B and the fourth distal through hole 64A are located at two opposite sides of the second plane P2, respectively, and equidistant from the second plane P2. Thus, the section of each of the driving wire 43 and 44 in the second pair of driving wires 48 routed through the proximal joint assembly 10 and the section of the same driving wire routed through the distal joint assembly 20 are located at two opposite sides of the second plane P2, respectively, and equidistant from the second plane P2. The above arrangement ensures that when the parallel motion mechanism 130 swings along the second plane P2, the increase (decrease) amount in length of each of the driving wire 43 and 44 routed through the proximal joint assembly 10 is equal to the decrease (increase) amount in length of the same driving wire routed through the distal joint assembly 20. Therefore, the second pair of driving wires 48, which controls the parallel motion mechanism 130 to swing in the first plane P1, is decoupled from the swing of the parallel motion mechanism 130 along the second plane P2. That is, the swing of the parallel motion mechanism 130 along the second plane P2 causes no change in length of the second pair of driving wires 48.

Optionally, the third proximal through hole 63B and the fourth proximal through hole 64B are located at two opposite sides of the second plane P2, respectively, and thus the third distal through hole 63A and the fourth distal through hole 64A are located at two opposite sides of the second plane P2, respectively. Therefore, the section of the third driving wire 43 routed through the proximal joint assembly 10 and the section of the fourth driving wire 44 routed through the proximal joint assembly 10 are located at two opposite sides of the second plane P2, respectively. The section of the third driving wire 43 routed through the distal joint assembly 20 and the section of the fourth driving wire 44 routed through the distal joint assembly 20 are located at two opposite sides of the second plane P2, respectively. The above arrangement allows the third driving wire 43 and the fourth driving wire 44 to independently control the parallel motion mechanism 130 to swing in opposite directions along the first plane P1, respectively.

Furthermore, optionally, the third proximal through hole 63B and the fourth proximal through hole 64B are equidistant from the second plane P2, and thus the third distal through hole 63A and the fourth distal through hole 64A are equidistant from the second plane P2. Therefore, the section of the third driving wire 43 routed through the proximal joint assembly 10 and the section of the fourth driving wire 44 routed through the proximal joint assembly 10 are equidistant from the second plane P2, and the section of the third driving wire 43 routed through the distal joint assembly 20 and the section of the fourth driving wire 44 routed through the distal joint assembly 20 are equidistant from the second plane P2. The above arrangement ensures that the driving forces required for the third driving wire 43 and the fourth driving wire 44 are equal.

Furthermore, optionally, the third proximal through hole 63B and the fourth proximal through hole 64B are arranged, for example, with 180-degree rotational symmetry about the central axis PC. The third distal through hole 63A and the fourth distal through hole 64A are arranged, for example, with 180-degree rotational symmetry about the central axis PC. The third proximal through hole 63B and the third distal through hole 63A are located at a same side of the first plane P1, and equidistant from the first plane P1. The fourth proximal through hole 64B and the fourth distal through hole 64A are located at a same side of the first plane P1, and equidistant from the first plane P1. The third proximal through hole 63B and the fourth proximal through hole 64B are located at two opposite sides of the first plane P1, respectively. The third distal through hole 63A and the fourth distal through hole 64A are located at two opposite sides of the first plane P1, respectively. As shown in FIGS. 7, 9, and 12, the third driving wire 43 and the fourth driving wire 44 are routed at two opposite sides of the first plane P1, respectively. Furthermore, each of the third driving wire 43 and the fourth driving wire 44 is routed parallel to the first plane P1, and the route of each of the third driving wire 43 and the fourth driving wire 44 between the third distal joint portion 23 and the third proximal joint portion 13 is straight. Therefore, the third driving wire 43 and the fourth driving wire 44, which are routed between the third distal joint portion 23 and the third proximal joint portion 13, are arranged with 180-degree rotational symmetry about the central axis PC. The above arrangement causes the lengths of the third driving wire 43 and the fourth driving wire 44 to be equal when the parallel motion mechanism 130 is in the neutral state, which may simplify the rear-end transmission device 150.

As shown in FIG. 8, the projections of the two driving wires 41 and 42 of the first pair of driving wires 47 onto the second plane P2 or other planes parallel to the second plane P2 intersect with each other. The projections of the two driving wires 43 and 44 of the second pair of driving wires 48 onto the second plane P2 or other planes parallel to the second plane P2 are parallel to each other (as shown in FIG. 8, the second plane P2 is parallel to the paper surface). As shown in FIG. 9, the projections of the two driving wires 41 and 42 of the first pair of driving wires 47 onto the first plane P1 or other planes parallel to the first plane P1 are parallel to each other. The projections of the two driving wires 43 and 44 of the second pair of driving wires 48 onto the first plane P1 or other planes parallel to the first plane P1 intersect with each other (as shown in FIG. 9, the first plane P1 is parallel to the paper surface). FIG. 8

As shown in FIGS. 10 and 11, optionally, when the parallel motion mechanism 130 is applied to an electrosurgical instrument, it is also necessary to reserve a space for the electric cable (not shown) of the end effector 110 to pass through, so that the driving wires 40 and the electric cable do not interfere with each other. Thus, each driving wire 40 has a section routed straightly between the third distal joint portion 23 and the third proximal joint portion 13, and the sections of the driving wires 40 are routed along different straight lines on a hyperboloid of one sheet. The principal axis of the hyperboloids of one sheet coincides with the central axis PC of the parallel motion mechanism 130. Due to the characteristics of the hyperboloid of one sheet, on the one hand, a space can be reserved for the electric cable to pass through (FIG. 12 shows the narrowest cross-section of the hyperboloid of one sheet). On the other hand, the driving wires 40 are constrained on the hyperboloid of one sheet when the driving wires 40 move, which will not interfere with the electric cable. Correspondingly, each of the first proximal joint portion 11, the second proximal joint portion 12, the third proximal joint portion 13, the third distal joint portion 23, the second distal joint portion 22, and the first distal joint portion 21 defines a cable passage 92 at a central position thereof for the electric cable to pass through. The electric cable is electrically coupled to the end effector 110 to supply power to the end effector 110. When the parallel motion mechanism 130 is in the neutral state, the electric cable is routed along the central axis PC.

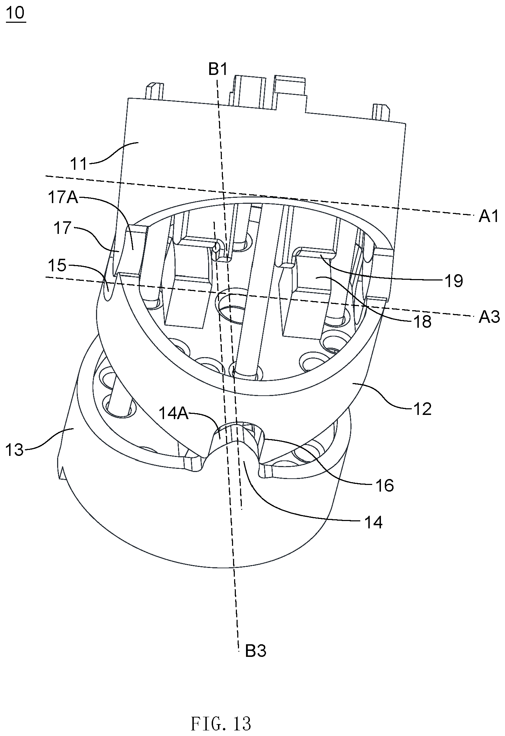

In the present disclosure, each joint of the proximal joint assembly 10 and the distal joint assembly 20 is designed as a rolling joint. Referring to FIG. 13, the following briefly describes the motion mechanism of the rolling joint and the schematic structure for realizing the motion mechanism, by taking the proximal joint assembly 10 as an example.

As shown in FIG. 13, each of two opposite sides of the second proximal joint portion 12 defines a first engagement recess 15. Each of two opposite sides of the first proximal joint portion 11 is provided with a first tooth 17. The first tooth 17 is received and held in the first engagement recess 15. When relative rotation of the first proximal joint portion 11 and the second proximal joint portion 12 occurs, the first tooth 17 is rotated within the first engagement recess 15, and edges defining an opening of the first engagement recess 15 are kept in contact with edges 17A of the first tooth 17.

Meanwhile, the first proximal joint portion 11 is provided with a first rolling surface 19, and the second proximal joint portion 12 is provided with a second rolling surface 18. When relative rotation of the first proximal joint portion 11 and the second proximal joint portion 12 occurs, the first rolling surface 19 and the second rolling surface 18 are kept in contact with each other and roll relative to each other. Specifically, each of the first rolling surface 19 and the second rolling surface 18 is constructed as an arc surface. The axis of the arc of the first rolling surface 19 is a first proximal axis A1. The axis of the arc of the second rolling surface 18 is a third proximal axis A3. The third proximal axis A3 is parallel to the first proximal axis A1 and maintains a constant distance from the first proximal axis A1. Each of the first proximal axis A1 and the third proximal axis A3 is perpendicular to the second plane P2.

By designing the shape of the surface of first tooth 17A and the engagement between the first tooth 17 and the first engagement recess 15, when the first tooth 17 is rotated within the first engagement recess 15, there is also a pure and rigid rolling (without relative sliding) between the first rolling surface 19 and the second rolling surface 18. When the first rolling surface 19 rigidly and purely rolls on the second rolling surface 18 in a first rotation direction, the first rolling surface 19 (or any part of the first proximal joint portion 11) revolves about the third proximal axis A3 in the first rotation direction by a first angle, while simultaneously rotating about the first proximal axis A1 in the first rotation direction by the first angle. Thus, relative rotation (swing) between the first proximal joint portion 11 and the second proximal joint portion 12 is realized.

Similar to the relative rotation of the first proximal joint portion 11 and the second proximal joint portion 12, the relative rotation of the second proximal joint portion 12 and the third proximal joint portion 13 is also achieved through rigid and pure rolling between some components. For example, the third joint portion 13 is provided with a second tooth 14, and the second proximal joint portion 12 is provided with a second engagement recess 16 for receiving and holding the second tooth 14. Meanwhile, the second proximal joint portion 12 is provided with an arc fourth rolling surface (not shown), and the third proximal joint portion 13 is provided with an arc third rolling surface (not shown) corresponding to the fourth rolling surface. The third rolling surface cooperates with the fourth rolling surface to realize rigid and pure rolling. The axis of the arc of the fourth rolling surface is a second proximal axis B1 (see FIGS. 3 to 8 and 10 to 12), and the axis of the arc of the third rolling surface is a fourth proximal axis B3. The second proximal axis B1 and the fourth proximal axis B3 are parallel, and the distance between the second proximal axis B1 and the fourth proximal axis B3 remains constant. Each of the second proximal axis B1 and the fourth proximal axis B3 is perpendicular to the first plane P1. By designing the shape of the surface of second tooth 14A and the engagement between the second tooth 14 and the second engagement recess 16, when the second tooth 14 is rotated within the second engagement recess 16, there is also a pure and rigid rolling (without relative sliding) between the third rolling surface and the fourth rolling surface.

The motion mechanism of relative rotation (swing) of the third proximal joint portion 13 and the second proximal joint portion 12 is similar to the motion mechanism of relative rotation (swing) of the first proximal joint portion 11 and the second proximal joint portion 12 mentioned above, and will not be further repeated.

The first engagement recess 15 and the second engagement recess 16 are arranged, for example, at interval of 90 degrees in a circumferential direction, so that the first proximal axis A1 and the second proximal axis B1 are perpendicular to each other.

Similarly, the distal joint assembly 20 may also have a same motion mechanism as that of the proximal joint assembly 10, and thus may be designed to have a same structure, which will not be further repeated.

In addition to the structure shown in FIG. 13, there may be other implementations for the structure that realizes the motion mechanism of the pivot joint. For example, the rolling surfaces of two adjacent joint portions may be omitted, and a connection rod may be provided between the two joint portions. The connection rod has two ends rotatably coupled to the two joint portions, respectively. For example, the surfaces of tooth and the engagement recesses of two adjacent joint portions may also be replaced with gear pairs that are engaged with each other.

The processes and steps described in all the embodiments are examples. Unless adverse effects occur, various operations may be performed in a sequence different from the process mentioned above. The steps in the above process may also be added, merged, or reduced according to actual needs.

When interpreting the scope of the present disclosure, the term "include" and its derivatives used herein indicate open-ended inclusion that specify the presence of some features, elements, components, groups, entities, and/or steps, and such terms do not exclude the presence of other unrecorded features, elements, components, groups, entities, and/or steps. The concept also applies to words with similar meanings, such as the terms "comprise", "provided with" and their derivatives.

The term "attached" or "attach" used herein encompasses a construction where an element is directly fixed to another element, a construction where an element is indirectly fixed to another element by securing the element to an intermediate component and securing the intermediate component to another element, and a construction where an element is integrally formed with another element which also means an element is essentially a part of another element. The above definition also applies to words with similar meanings, such as "connect", "link", "couple", "install" "adhere", "fix", and their derivatives. Finally, degree terms such as "basically", "substantially", and "approximately" used herein indicate the amount of deviation that modified by the term will not significantly change the final result.

Unless otherwise defined, the technical and scientific terms used in the present disclosure have the same meaning as commonly understood by those skilled in the art. The terms used in the present disclosure are for the purpose of describing specific embodiments, but not intended to limit the present disclosure. The features described in one embodiment in the present disclosure may be applied to another embodiment, either individually or in combination with other features, unless such feature is not applicable in other embodiment or otherwise specified.

The present disclosure has been described through the above embodiments. It should be understood that the embodiments are for illustration and explanation purposes, but not intended to limit the disclosure to the described embodiments. Furthermore, those skilled in the art will appreciate that the present disclosure is not limited to the above embodiments. Various modifications and changes may be made based on the teachings of the present disclosure, which fall within the scope claimed by the present disclosure.

## Claims

1. A parallel motion mechanism of a surgical instrument, comprising:
a proximal joint assembly comprising a first proximal joint portion, a second proximal joint portion, and a third proximal joint portion coupled in sequence, the third proximal joint portion being configured to swing along a first plane relative to the second proximal joint portion, the second proximal joint portion being configured to swing along a second plane relative to the first proximal joint portion, and the first plane and the second plane intersecting with each other at a central axis of the parallel motion mechanism;
a distal joint assembly being configured to couple to an end effector, and comprising a first distal joint portion, a second distal joint portion, and a third distal joint portion coupled in sequence, the third distal joint portion coupled to the third proximal joint portion and fixed relative to the third proximal joint portion, the second distal joint portion being configured to swing along the first plane relative to the third distal joint portion, and the first distal joint portion being configured to swing along the second plane relative to the second distal joint portion;
a plurality of constraint wires being configured to maintain an orientation of the first distal joint assembly relative to the first proximal joint, each of the plurality of constraint wires having an end fixed to the first proximal joint portion and another end fixed to the first distal joint portion; and
a plurality of driving wires being configured to actuate the distal joint assembly, each of the plurality of driving wires having an end fixed to the first distal joint portion and another end configured to couple to a rear-end transmission device, each of the plurality of driving wires having a portion routed through the parallel motion mechanism, and a sum of lengths of the portions of the plurality of driving wires remaining unchanged.

2. The parallel motion mechanism according to claim 1, wherein each of the plurality of driving wires has a section routed through the proximal joint assembly and a section routed through the distal joint assembly, both of the sections of each of the plurality of driving wires are routed parallel to the central axis when the parallel motion mechanism is in a neutral state, and the plurality of driving wires comprises:
a first pair of driving wires, wherein a section of each driving wire in the first pair of driving wires routed through the proximal joint assembly and a section of the driving wire routed through the distal joint assembly are located at two opposite sides of the first plane, respectively, and are equidistant from the first plane; and
a second pair of driving wires, wherein a section of each driving wire in the second pair of driving wires routed through the proximal joint assembly and a section of the driving wire routed through the distal joint assembly are located at two opposite sides of the second plane, respectively, and are equidistant from the second plane.

3. The parallel motion mechanism according to claim 2, wherein the first pair of driving wires comprises a first driving wire and a second driving wire, the first driving wire and the second driving wire are configured to drive the first distal joint portion to swing along the second plane relative to the second distal joint portion, and a section of the first driving wire routed through the proximal joint assembly and a section of the second driving wire routed through the proximal joint assembly are located at two opposite sides of the first plane, respectively.

4. The parallel motion mechanism according to claim 3, wherein the section of the first driving wire routed through the proximal joint assembly and the section of the second driving wire routed through the proximal joint assembly are equidistant from the first plane.

5. The parallel motion mechanism according to claim 3 or 4, wherein each of the first driving wire and the second driving wire has a section routed straightly between the third distal joint portion and the third proximal joint portion and parallel to the second plane, and the sections of the first driving wire and the second driving wire are located at two opposite sides of the second plane, respectively.

6. The parallel motion mechanism according to any one of claims 2 to 5, wherein the second pair of driving wires comprises a third driving wire and a fourth driving wire, the third driving wire and the fourth driving wire are configured to drive the second distal joint portion to swing along the first plane relative to the third distal joint portion, and a section of the third driving wire routed through the proximal joint assembly and a section of the fourth driving wire routed through the proximal joint assembly are located at two opposite sides of the second plane, respectively.

7. The parallel motion mechanism according to claim 6, wherein the section of the third driving wire routed through the proximal joint assembly and the section of the fourth driving wire routed through the proximal joint assembly are equidistant from the second plane.

8. The parallel motion mechanism according to claim 6 or 7, wherein the third driving wire and the fourth driving wire each is parallel to the first plane, and the third driving wire and the fourth driving wire are located at two opposite sides of the first plane, respectively.

9. The parallel motion mechanism according to any one of claims 1 to 8, wherein each of the plurality of driving wires has a section routed straightly between the third distal joint portion and the third proximal joint portion, the sections of the plurality of driving wires are routed along different straight lines on a hyperboloid of one sheet, and a principal axis of the hyperboloid of one sheet coincides with the central axis of the parallel motion mechanism.

10. The parallel motion mechanism according to any one of claims 1 to 9, wherein the plurality of constraint wires comprises at least two pairs of constraint wires; the at least two pairs of constraint wires are routed parallel to the central axis, and are symmetrical with respect to each of the first plane and the second plane when the parallel motion mechanism is in a neutral state.

11. The parallel motion mechanism according to any one of claims 1 to 10, further comprising a sleeve, wherein the sleeve has two ends coupled to the third distal joint portion and the third proximal joint portion, respectively, and each of the plurality of driving wires and the plurality of constraint wires is routed through an interior of the sleeve.

12. A surgical instrument, comprising:
a parallel motion mechanism according to any one of claims 1 to 11;
an end effector coupled to the distal joint assembly of the parallel motion mechanism; and
a rear-end transmission device, each of the plurality of driving wires having a rear end coupled to the rear-end transmission device.

13. The surgical instrument according to claim 12, wherein the end effector is a hook, a spatula, a needle, a clamp, or a pair of scissors.

14. The surgical instrument according to claim 12 or 13, further comprising an end joint assembly, wherein the end effector is coupled to the distal joint assembly through the end joint assembly.

15. The surgical instrument according to claim 14, wherein the end joint assembly comprises a yaw joint base and a pitch joint base, the end effector has a proximal end coupled to a distal end of the yaw joint base and rotatable about a yaw axis relative to the yaw joint base, and the yaw joint base has a proximal end coupled to a distal end of the pitch joint base and rotatable about the yaw axis relative to the pitch joint base.

16. The surgical instrument according to claim 15, wherein the proximal end of the end effector is rotatably coupled to the distal end of the yaw joint base through a first clevis pin, and/or the proximal end of the yaw joint base is rotatably coupled to the distal end of the pitch joint base through a second clevis pin.

17. The surgical instrument according to claim 16, wherein the end effector comprises a first jaw and a second jaw, each of the first jaw and the second jaw has a proximal end pivotably coupled to the yaw joint base through the first clevis pin, and the first jaw and the second jaw are separately pivotable relative to the yaw joint base about the first clevis pin.

18. The surgical instrument according to any one of claims 14 to 17, further comprising at least one pair of end joint driving wires, wherein the at least one pair of end joint driving wires is configured to acuate the end joint assembly relative to the distal joint assembly, thereby moving the end effector relative to the distal joint assembly, the at least one pair of end driving wires is routed at the first plane and/or the second plane and parallel to the central axis when the parallel motion mechanism is in a neutral state, and each of the at least one pair of end driving wires has an end coupled to the rear-end transmission device.

19. The surgical instrument according to any one of claims 12 to 18, further comprising an electric cable, wherein the electric cable is electrically coupled to the end effector, and the electric cable is routed along the central axis when the parallel motion mechanism is in a neutral state.

20. A surgical robot, comprising:
a robotic arm equipped with an instrument transmission device; and
a surgical instrument according to any one of claims 12 to 19, the surgical instrument removably coupled to the robotic arm, and the instrument transmission device being engaged with the rear-end transmission device to actuate the rear-end transmission device to pull in or release the plurality of driving wires.
